# EUROPÄISCHE PATENTSCHRIFT

(11) **EP 0 563 613 B1**
(45) Veröffentlichungstag und Bekanntmachung des Hinweises auf die Patenterteilung: **20.08.1997**
(21) Anmeldenummer: 93103490.4
(22) Anmeldetag: 04.03.1993
(51) Int. Cl.: G01N 27/12, G01N 27/14

(54) **Anordnung zur Bestimmung eines Gaspartialdruckes in einem Gasgemisch**
Device for determining the partial pressure of gases in a gas mixture
Dispositif de détermination de la pression partielle de gaz dans un mélange gazeux

(30) Priorität: 30.03.1992 DE 4210398
(43) Veröffentlichungstag der Anmeldung: 06.10.1993
(73) Patentinhaber: SIEMENS AKTIENGESELLSCHAFT, 80333 München (DE)
(72) Erfinder: Gerblinger, Josef, Dr., W-8900 Ausburg (DE); Meixner, Hans, Prof., W-8013 Haar (DE)

(56) Entgegenhaltungen:
- EP-A- 0 001 512
- EP-A- 0 014 092
- EP-A- 0 056 752

## Beschreibung

Die steigenden Umweltbelastungen (z.B. Treibhauseffekt) durch Verbrennungsprodukte (z.B. CO, CO₂, CHₓ oder NOₓ) haben in den letzten Jahren zu einer immer stärker werdenden Forderung nach einer Reduzierung der Schadstoffemissionen bei Verbrennungsprozessen aller Art geführt.

Aus der Notwendigkeit heraus, die Verbrennungsprozesse zu optimieren, erwächst die Forderung nach Sensoren, die die Verbrennungsprodukte dieser Prozesse mit hinreichender Schnelligkeit und Genauigkeit detektieren können. Im besonderen Maße besteht die Notwendigkeit, Sauerstoffpartialdrücke in Gasgemischen zu detektieren, um so bei Verbrennungsprozessen stöchiometrische Gemische einstellen oder Einhalten zu können. Besonders schadstoffintensive, häufige Verbrennungsprozesse treten beim Betrieb von Verbrennungsmotoren auf. In Kraftfahrzeugen z.B. werden zur Detektion von Sauerstoffpartialdrücken Ytrium-stabilisierte Zirkonoxid ZrO₂ Sensoren verwendet. Diese benötigen für ihren Betrieb ein Sauerstoff Referenzvolumen, welches die Miniaturisierung der Sonden begrenzt/1/.

Im Unterschied zu diesen potentiometrischen Sonden können auch resistive Sauerstoffsensoren eingesetzt werden. Bei diesen Sonden ändert sich die Leitfähigkeit des sensitiven Materials entsprechend dem Sauerstoffpartialdruck der Umgebung des Sensors/2/. Bei genügend hohen Temperaturen stellt sich ein Gleichgewicht zwischen den im Metalloxidgitter, das für die Sensoren Verwendung findet, vorhandenen Sauerstoffleerstellen und den Gasmolekülen der MR/The - 27.3.1992 Umgebung ein. Wird z.B. das Angebot von Sauerstoffmolekülen an der Oberfläche des sensitiven Materials verringert, so diffundieren Sauerstoffionen aus dem Metalloxidgitter an die Oberfläche, verbinden sich zu Sauerstoffmolekülen und verlassen schließlich das Gitter.

Dieser Vorgang dauert so lange, bis sich ein chemisches Gleichgewicht zwischen den Konzentrationen der Sauerstoffionen im Gitter und den Sauerstoffmolekülen der Umgebung einstellt. Beim Verlassen der Gitterplätze bleiben Gitterelektronen zurück, die zu einem Anstieg der Elektronenleitung führen. Bei einem Anwachsen der Konzentration von Sauerstoffmolekülen in der Sensorumgebung nimmt das sensitive Material Sauerstoffionen auf. In diesem Fall verringert sich die zur elektrischen Leitfähigkeit beitragende Anzahl von Elektronen. Da die geschilderten Vorgänge reversibel sind, kann jedem Sauerstoffpartialdruck der Sensorumgebung eine bestimmte Leitfähigkeit des sensitiven Materials zugeordnet werden/3/. Resistive Sauerstoffsensoren benötigen im Gegensatz zu potentiometrischen Sauerstoffsensoren keine Referenzatmosphäre. Es steht einer Miniaturisierung dieser Sensoren also nichts im Wege/4/. Bisher werden vor allem Titanoxid TiO₂ und Zinnoxid SnO₂ als Sensormaterialien für resistive Sauerstoffsensoren verwendet /5,6/. Beide Materialien weisen aber eine Reihe von Nachteilen auf, die den gewünschten Einsatz im Abgastrakt eines Kraftfahrzeuges nicht erlauben. So sind Sensoren aus SnO₂ nur bis Temperaturen von maximal 500°C sinnvoll einsetzbar und besitzen in diesem Temperaturbereich zusätzlich in erhöhtem Maße Querempfindlichkeiten gegenüber CO, HC und H₂. TiO₂ Sensoren sind für den gewünschten Anwendungsfall ebenfalls nicht geeignet, da dieses Material nur bis maximal 950°C einsetzbar ist.

In der EP-A-0 014 092 ist eine Anordnung zur Bestimmung des Sauerstoffpartialdruckes in einem Gasgemisch beschrieben, die aus zwei in Serie geschalteten Sensorelementen aus TiO₂ besteht. Das eine Sensorelement ist mehr sauerstoffempfindlich, das andere Sensorelement ist mehr temperaturempfindlich. Erreicht wird diese unterschiedliche Empfindlichkeit durch eine unterschiedliche Materialdichte des verwendeten TiO₂.

Die dieser Erfindung zugrundeliegende Aufgabe besteht darin, eine weitere Anordnung anzugeben, mit der Partialdrücke von Gasen in Gasgemischen bestimmt werden können.

Diese Augabe wird gemäß den Merkmalen des Patentanspruchs 1 gelöst.

Alle übrigen Weiterbildungen der Erfindung ergeben sich aus den Unteransprüchen.

Ein besonderer Vorteil der erfindungsgemäßen Anordnung besteht darin, daß der zweite Gassensor aus demselben Material besteht wie der erste Gassensor und durch eine geeignete Maßnahme vom Sauerstoffpartialdruck des Gasgemisches unabhängig gemacht worden ist. Es ist durch diese Maßnahme sichergestellt, daß dieser zweite Gassensor dasselbe temperaturabhängige Leitfähigkeitsverhalten aufweist wie der erste Gassensor. Somit kann mit großer Genauigkeit aus den beiden Signalen, die an den Gassensoren gemessen werden können, ein Auswertesignal ermittelt werden, das dem Gaspartialdruck des zu bestimmenden Gases aus dem Gasgemisch proportional ist. Es kann auf einen zusätzlichen Temperatursensor für die Sensorheizung verzichtet werden, da der zweite Gassensor als solcher dienen kann. Besonders vorteilhaft bei der erfindungsgemäßen Anordnung ist es auch, daß mit ihr Sauerstoffpartialdrücke bestimmt werden können, denen eine große Rolle bei Verbrennungsprozessen zukommt.

Weiterhin vorteilhaft ist es, Sensoren aus Metalloxiden einzusetzen, da es viele Metalloxide gibt, die Sauerstoff interstitiell gelöst enthalten und somit als Sensormaterial in Frage kommen.

Besonders geeignet für den Einsatz als Sensormaterialien in der erfindungsgemäßen Anordnung sind SrTiO₃, TiO₂ oder CeO₂, da sie aufgrund ihrer Materialeigenschaften ein besonders günstiges Detektionsverhalten für Sauerstoffpartialdrücke aufweisen.

Besonders günstig für den Einsatz in der erfindungsgemäßen Anordnung sind Gold, Calzium oder Yttrium als Dotierungsmaterialien. Sie sind besonders geeignet das Ansprechverhalten der Sensormaterialien auf Partialdruckänderungen, auch schon bei niedrigen Dotierungskonzentrationen zu neutralisieren. So wird sichergestellt, daß das temperaturabhängige Leitfähigkeitsverhalten der Sensormaterialien durch die Dotierungsmaßnahmen nicht beeinträchtigt wird.

Günstig ist es in der erfindungsgemäßen Anordnung besonders SrTiO₃ mit Gold zu dotieren, da schon geringe Golddotierungskonzentrationen ausreichen, um das SrTiO₃ als Sensormaterial für Partialdruckänderungen des Umgebungsgases unempfindlich zu machen.

Weiterhin günstig am Einsatz in der erfindungsgemäßen Anordnung sind Sensoren aus CeO₂, die mit Yttrium oder Calzium dotiert sind, da diese Materialien geeignet sind das Leitfähigkeitsverhalten in Bezug auf die Partialdruckänderungen des Umgebungsgases auch schon in geringen Konzentrationen zu neutralisieren.

Weiterhin vorteilhaft ist der Einsatz der erfindungsgemäßen Anordnung in einem Verfahren, bei dem die Atemfunktion von Lebewesen überwacht wird. Das schnelle Ansprechverhalten der Sensoren ist besonders geeignet, kritische Situationen in der Atemfunktion von Lebewesen herauszufinden und geeignete Maßnahmen einzuleiten.

Weiterhin vorteilhaft bei der erfindungsgemäßen Anordnung ist es beide Gassensoren auf einem Substrat aufzubringen, um die Abmessung der Anordnung zu verkleinern.

Vorteilhaft ist es, in der erfindungsgemäßen Anordnung ein Substrat aus Al₂O₃ zu verwenden, da es in Bezug auf seine Temperatureigenschaften besonders für den Einsatz bei hohen Temperaturen geeignet ist.

Günstig ist auch der Einsatz des Sputterverfahrens zur Aufbringung der Sensorgeometrie auf das Subtrat, da damit das Meßverhalten der beiden Gassensoren genau eingestellt werden kann.

Vorteilhaft ist auch die Anwendung des Siebdruckverfahrens zur Aufbringung der Sensorgeometrie auf das Substrat, da es für die Massenproduktion besonders geeignet ist.

Im folgenden wird die erfindungsgemäße Anordnung durch Figuren weiter erläutert.
- Figur 1: zeigt ein Beispiel der erfindungsgemäßen Anordnung.
- Figur 2: zeigt ein Beispiel eines Sensoraufbaues der erfindungsgemäßen Anorndung.
- In Figur 3: ist die Leitfähigkeit der Sensoren in Abhängigkeit des Sauerstoffpartialdrucks bei unterschiedlichen Temperaturen dargestellt.

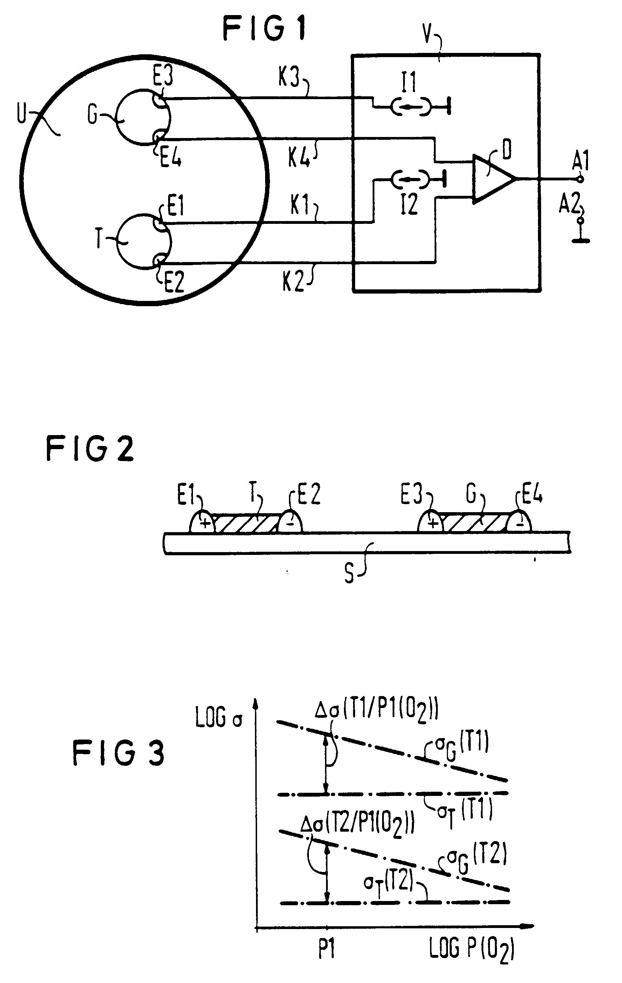

In Figur 1 ist ein Beispiel der erfindungsgemäßen Anordnung dargestellt. Sie zeigt ein Gasgemisch U, einen ersten Gassensor G und einen Temperatursensor T. Der erste Gassensor G weist Anschlüsse E3 und E4 auf. Der Temperatursensor T weist Anschlüsse E1 und E2 auf. Am ersten Gassensor kann ein Signal S1 gemessen werden und am zweiten Gassensor wird in Abhängigkeit der Temperatur ein Signal S2 gemessen.

Über elektrisch leitende Verbindungsleitungen K1 bis K4 die gleich nummeriert sind wie die Anschlüsse E, mit denen sie verbunden sind, sind der erste Gassensor G und der zweite Gassensor T mit einer nachgeschalteten Verarbeitungseinheit V verbunden. In der nachgeschalteten Verarbeitungseinheit V befinden sich eine Konstantstromquelle I1, die beispielsweise einen Strom von 100 Mikroampere liefert. Diese Konstantstromquelle I1 versorgt den ersten Gassensor G mit einem Konstantstrom. Bei einer Widerstandsänderung des ersten Gassensors findet an ihm ein Spannungsabfall statt. Eine zweite Konstantstromquelle I2 innerhalb der Verarbeitungseinheit V, ist mit dem zweiten Gassensor T verbunden, der ein nur von der Temperatur abhängiges Signal liefert. Im Falle einer Widerstandsänderung am Gassensor T findet dort ein Spannungsabfall statt. Diese beiden Spannungsabfälle werden einem Differenzverstärker D zugeführt, dieser subtrahiert diese beiden Spannungsabfälle und liefert an den Ausgängen A1 und A2 ein dem Partialdruck eines Gases innerhalb des Gasgemisches proportionales Ausgangssignal. Hier wird der Sauerstoffpartialdruck bestimmt. Der Differenzverstärker D kann beispielsweise aus einem Operationsverstärker aufgebaut sein. Dieser Operationsverstärker ist beispielsweise beschaltet wie es in U. Tietze, CH. Schenk,"Halbleiterschaltungstechnik" (9.Auflage) unter Subtrahierer beschrieben ist.

Die Widerstandsänderung des ersten Gassensors, bzw. die Leitfähigkeitsänderung des ersten Gassensors unter Einfluß von Sauerstoffpartialdurckänderungen des Gasgemisches U läßt sich wie folgt erklären: Im Falle einer Partialdruckzunahme des Sauerstoffes versuchen Sauerstoffmoleküle in das Gitter, aus dem der erste Gassensor aufgebaut ist, einzudringen. Sie spalten sich zu Ionen auf und diffundieren in das Gitter ein. Im Gitter binden diese Sauerstoffionen Elektronen an sich und das Gitter verarmt so langsam an Elektronen. Die Leitfähigkeit nimmt ab. Das heißt der Widerstand wird größer. Mit zunehmender Eindiffusion in das Gitter wird ein anderer Leitungseffekt dominierend. Die Defektelektronenleitung, d.h. Löcherleitung. Die Leitfähigkeit des ersten Gassensors G nimmt wieder zu, bzw. der Ohm'sche Widerstand nimmt wieder ab. Diese beschriebenen Vorgänge sind reversibel. Falls also eine Partialdruckabnahme im Gasgemisch U stattfindet, so diffundieren die Sauerstoffionen wieder aus dem Gitter des ersten Gassensors heraus und bilden Sauerstoffmoleküle. Dabei hinterlassen sie im Gitter die Elektronen, die sie vorher an sich gebunden hatten und die Leitfähigkeit des Sensormaterials nimmt wieder zu, bzw. der Ohm' sche Widerstand nimmt ab. Diese Änderungen können mit der Verarbeitungseinheit V festgestellt werden und führen zu einer Änderung im Ausgangssignal, daß an den Anschlüssen A1 und A2 abgegeben wird.

Figur 2 zeigt die erfindungsgemäße Anordnung am Beispiel eines Sensoraufbaues. Es sind dargestellt Elektroden E1 bis E4 und ein Substrat S, ein zweiter Gassensor T und ein erster Gassensor G. Die Gassensoren können beispielsweise aus SrTiO₃ oder TiO₂, oder CeO₂ ausgeführt sein. Die einzelnen Bestandteile der erfindungsgemäßen Anordnung können beispielsweise durch Sputtern oder Siebdruck aufgebracht sein. An den Elektroden E1 und E2 kann analog zur Figur 1 das Signal S2 abgegriffen werden. An den Elektroden E3 und E4 kann wie in Figur 1 am ersten Gassensor G das Signal S1 gemessen werden. Als Substrat in diesem Aufbau dient beispielsweise Al₂O₃. Wie man besonders vorteilhaft aus Figur 2 erkennen kann, ist für den zweiten Gassensor T keine zusätzliche Abschirmungsmaßnahme vom Gasgemisch erforderlich. Dies wird dadurch erreicht, daß der zweite Gassensor nicht auf Partialdruckänderungen reagiert, weil er dotiert ist und somit seine Leitfähigkeit nur in Abhängigkeit der Temperatur und nicht in Abhängigkeit vom sauerstoffpartialdruck ändert. Dieser zweite Gassensor T kann deshalb auch als Temperatursensor für die Sensorheizung verwendet werden. Unsicherheiten im Isolationsverhalten, wie sie bei der Passivierung mit Nitrid- Verbindungen auftreten können, kommen nicht vor.

In Figur 3 ist die Leitfähigkeit Gassensor G, T und der Sauerstoffpartialdruck in Abhängigkeit der Temperatur dargestellt. Es werden dargestellt das Leitfähigkeitsverhalten des ersten Gassensors σ _{G} und des zweiten Gassensors σ_{T} bei zwei verschiedenen Temperaturen T1 und T2. Auf der horizontalen Achse ist der Logarithmus des Sauerstoffpartialdrucks des Gasgemisches U aufgetragen und auf der vertikalen Achse der Logarithmus der Leitfähigkeit σ. In diesem Fall ist T1 größer als T2, und wie man erkennen kann, steigt die Leitfähigkeit mit zunehmender Temperatur an. Bei der Temperatur T2 weist die Leitfähigkeit des zweiten Gassensors T keine Abhängigkeit vom Sauerstoffpartialdruck auf. Das sieht man daran, daß σ_{T}(T2) über den gesamten Druckbereich konstant bleibt. Deutlich ist erkennbar, daß der erste Gassensor G eine Abhängigkeit vom Sauerstoffpartialdruck aufweist. Dies erkennt man aus dem Verlauf der Geraden σ_{G}(T2). Auch bei der Temperatur T1 kann man erkennen, daß der zweite Gassensor T keine Abhängigkeit vom Sauerstoffpartialdruck aufweist. Erreicht wurde dieser Sachverhalt durch Dotierung mit einem Material wie beispielsweise Gold. Dies ergibt sich aus der Geraden σ_{T}(T1). Sie verläuft über den gesamten Druckbereich konstant. Der erste Gassensor weist auch bei der höheren Temperatur T1 eine Abhängigkeit vom Sauerstoffpartialdruck des Gasgemisches U auf, wie man dies am Verlauf der Geraden σ _{G}(T1) erkennen kann. Weiterhin ist zu bemerken, daß sich die Partialdruckabhängikeit des ersten Gassensors G bei der höheren Temperatur T1 durch eine Parallelverschiebung der Gerade bei der niederen Temperatur T2 ableiten läßt. Ebenso gilt dies für die Abhängigkeit des zweiten Gassensors bei T1 und T2. Die Geraden lassen sich auch hier durch Parallelverschiebung ineinander überführen. Deutlich kann man erkennen, daß der Betragsunterschied Δσ bei einem Umgebungsdruck P1 bei den Temperaturniveaus T1 und T2 gleichbleibt. Diese Bedingung ist besonders wichtig für die erfindungsgemäße Anordnung, da nur so direkt der Sauerstoffpartialdruck des Gasgemisches ermittelt werden kann, weil die Temperaturabhängigkeit durch die Differenzbildung zwischen dem Signal des zweiten Gassensors S2 und dem Signal des ersten Gassensors S1 bei beliebigen Temperaturen herausfällt.

### Literaturliste

/1/ Velacso, G. und Pribat, D.:Microionic Gas Sensors for Pollution and Energy Controllin the Consumer Market, Proc.2.Int.Meeting on Chemical Sensors, Bordeaux (1986), S.79-94
/2/ Kofstad, P.: Nonstoichiometry, Diffusion and Electrical Conductivity in Binary Metal Oxides Wiley & Sons, New York, Reprint Edition (1983)
/3/ Härdtl, K.H.: Ceramic Sensors Sci.Ceram, **14** (1988), S.73-88
/4/ Schönauer, U.: Dickschicht-Sauerstoffsensoren auf der Basis keramischer Halbleiter Tech-Mess., **56,6** (1989), S.260-263
/5/ Hoshino,K., Peterson, N.L. u. Wiley, C.I.: Diffusion and Point Defects in TiO₂ J.Phys.Chem.Solids, **46**, 12 (1985), S.1397 -1411
/6/ Göpel, W.: Solid-State Chemical Sensors: Atomistic Models and Research Trends Sensors and Actuators, **16** (1989), S.167-193

## Patentansprüche

1. Anordnung zur Bestimmung eines Gaspartialdruckes in einem Gasgemisch (U), bei der
a) ein erster Gassensor (G) aus einem ersten Material vorgesehen ist, das in Abhängigkeit des zu bestimmenden Gaspartialdruckes seine Leitfähigkeit bzw. seinen Ohm'schen Widerstand ändert, und ein erstes Meßsignal (S1) zur Bestimmung des Gaspartialdruckes gemessen wird,
b) ein zweiter Gassensor (T) aus demselben Material wie der erste Gassensor (G) vorgesehen ist, der den gleichen Umgebungsbedingungen unterliegt wie der erste Gassensor (G), und bei dem durch Dotierung mit einem zweiten Material sichergestellt ist, daß er seinen Ohm'schen Widerstand bzw. seine elektrische Leitfähigkeit nicht mehr in Abhängigkeit des Gaspartialdruckes des zu bestimmenden Gases ändert, und ein zweites Meßsignal (S2) gemessen wird,
c) sich ein dem zu bestimmenden Gaspartialdruck proportionales Auswertesignal unabhängig von der Temperatur des Gasgemisches dadurch ergibt, daß das erste und das zweite Meßsignal (S1, S2) voneinander subtrahiert werden.

2. Anordnung nach Anspruch 1, bei der der Sauerstoffpartialdruck eines Gasgemisches (U) bestimmt wird.

3. Anordnung nach einem der Ansprüche 1 oder 2, bei dem das Sensormaterial der Gassensoren (G, T) wenigstens ein Metalloxid ist.

4. Anordnung nach einem der Ansprüche 1 bis 3, bei der das Sensormaterial SrTiO₃, oder TiO₂, oder CeO₂ ist.

5. Anordnung nach einem der Ansprüche 1 bis 4, bei der das als Dotierungsmaterialien wenigstens Gold, Calzium oder Yttrium Verwendung finden.

6. Anordnung nach einem der Ansprüche 1 bis 5, bei der SrTiO₃ mit Gold dotiert wird.

7. Anordnung nach einem der Ansprüche 1 bis 5, bei der ClO₂ wenigstens mit Yttrium, oder Calzium dotiert wird.

8. Anordnung nach einem der Ansprüche 1 bis 7, bei der die Gassensoren (G, T) auf einem Substrat (S) aufgebracht sind.

9. Anordnung nach Anspruch 8, bei der das Substrat (S) Al₂O₃ ist.

10. Anordnung nach Anspruch 8, bei der die Aufbringung der Sensorgeometrie durch Sputtern erfolgt.

11. Anordnung nach Anspruch 8, bei der die Aufbringung der Sensorgeometrie durch Siebdruck erfolgt.

12. Verwendung der Anordnung nach einem der Ansprüche 1 bis 11, zur Überwachung der Atemfunktion eines Lebewesens, bei dem die durch den Atemvorgang bewirkte periodische Sauerstoffpartialdruckänderung der ausgeatmeten Luft ermittelt wird und Unregelmäßigkeiten der Periode ausgewertet werden.

## Claims

1. Arrangement for determining a gas partial pressure in a gas mixture (U), in which
a) a first gas sensor (G) is provided, made of a first material which changes its conductivity or its resistance as a function of the gas partial pressure to be determined, and a first measurement signal (S1) for determining the gas partial pressure is measured,
b) a second gas sensor (T) made of the same material as the first gas sensor (G) is provided, which is subjected to the same ambient conditions as the first gas sensor (G), and in which doping with a second material ensures that it no longer changes its resistance or its electrical conductivity as a function of the gas partial pressure of the gas to be determined, and a second measurement signal (S2) is measured,
c) an evaluation signal, proportional to the gas partial pressure to be determined, is produced, independently of the temperature of the gas mixture, by taking the difference between the first and second measurement signals (S1, S2).

2. Arrangement according to Claim 1, in which the oxygen partial pressure of a gas mixture (U) is determined.

3. Arrangement according to one of Claims 1 or 2, in which the sensor material of the gas sensors (G, T) is at least one metal oxide.

4. Arrangement according to one of Claims 1 to 3, in which the sensor material is SrTiO₃, or TiO₂, or CeO₂.

5. Arrangement according to one of Claims 1 to 4, in which at least gold, calcium or yttrium are used as dopants.

6. Arrangement according to one of Claims 1 to 5, in which SrTiO₃ is doped with gold.

7. Arrangement according to one of Claims 1 to 5, in which ClO₂ is doped at least with yttrium or calcium.

8. Arrangement according to one of Claims 1 to 7, in which the gas sensors (G, T) are applied to a substrate (S).

9. Arrangement according to Claim 8, in which the substrate (S) is Al₂O₃.

10. Arrangement according to Claim 8, in which the sensor geometry is applied by sputtering.

11. Arrangement according to Claim 8, in which the sensor geometry is applied by screen printing.

12. Use of the arrangement according to one of Claims 1 to 11 for monitoring the respiratory function of a living being, in which the periodic oxygen partial pressure change in the exhaled air, due to the breathing process, is determined and irregularities in the period are evaluated.

## Revendications

1. Dispositif pour déterminer une pression partielle de gaz dans un mélange gazeux (U), dans lequel
a) un premier détecteur de gaz (G) est composé d'un premier matériau dont la conductivité ou la résistance ohmique varie en fonction de la pression partielle de gaz à déterminer et un premier signal de mesure (S1) est mesuré pour déterminer la pression partielle de gaz,
b) un second détecteur de gaz (T) est composé du même matériau que le premier détecteur de gaz (G), qui est sujet aux mêmes conditions ambiantes que le premier détecteur de gaz (G) et pour lequel est assuré par dopage avec un second matériau que sa résistance ohmique ou sa conductivité électrique ne varie plus en fonction de la pression partielle de gaz du gaz à déterminer, et dans lequel un second signal de mesure (S2) est mesuré,
c) un signal d'évaluation proportionnel à la pression partielle de gaz à déterminer est obtenu indépendamment de la température du mélange gazeux par soustraction du premier et du second signal de mesure (S1, S2).

2. Dispositif selon la revendication 1, dans lequel la pression partielle d'oxygène d'un mélange gazeux (U) est déterminée.

3. Dispositif selon l'une des revendications 1 ou 2, dans lequel le matériau des détecteurs de gaz (G, T) est au moins un oxyde métallique.

4. Dispositif selon l'une des revendications 1 à 3, dans lequel le matériau des détecteurs est à base de SrTiO₃ ou TiO₂ ou CeO₂.

5. Dispositif selon l'une des revendications 1 à 4, dans lequel au moins de l'or, du calcium ou de l'yttrium sont utilisés comme matériaux de dopage.

6. Dispositif selon l'une des revendications 1 à 5, dans lequel SrTiO₃ est dopé avec de l'or.

7. Dispositif selon l'une des revendications 1 à 5, dans lequel ClO₂ est dopé au moins avec de l'yttrium ou du calcium.

8. Dispositif selon l'une des revendications 1 à 7, dans lequel les détecteurs de gaz (G, T) sont appliqués sur un substrat (S).

9. Dispositif selon la revendication 8, dans lequel le substrat (S) est du Al₂O₃.

10. Dispositif selon la revendication 8, dans lequel l'application de la géométrie des détecteurs est réalisée par pulvérisation.

11. Dispositif selon la revendication 8, dans lequel l'application de la géométrie des détecteurs est réalisée par sérigraphie.

12. Mise en oeuvre du dispositif selon l'une des revendications 1 à 11 pour surveiller la fonction respiratoire d'un être vivant, dans laquelle la variation périodique de la pression partielle d'oxygène de l'air expiré, variation provoquée par les mouvements respiratoires, est déterminée et des irrégularités de la période sont évaluées.
